# EUROPEAN PATENT SPECIFICATION

(11) **EP 1 659 987 B1**
(45) Date of publication and mention of the grant of the patent: **05.10.2011**
(21) Application number: 04780317.6
(22) Date of filing: 06.08.2004
(51) Int. Cl.: A61F 2/90

(54) **DEFORMABLE MEDICAL DEVICE WITHOUT MATERIAL DEFORMATION**
VERFORMBARES MEDIZINPRODUKT OHNE MATERIALVERFORMUNG
DISPOSITIF MEDICAL SE DEFORMANT SANS DEFORMATION DE MATIERE

(30) Priority: 11.08.2003 US 638819
(43) Date of publication of application: 31.05.2006
(73) Proprietor: Boston Scientific Limited, St. Michael, Barbados, West Indies (BB)
(72) Inventor: NEUENDORF, Rachel, San Francisco, CA 94107 (US); WEBER, Jan, Maple Grove, MN 55311 (US)
(74) Representative: Hauck Patent- und Rechtsanwälte
(86) International application number: PCT/US2004/025462
(87) International publication number: WO 2005/018500

(56) References cited:
- US-A1- 2002 111 671
- US-A1- 2002 188 345
- US-B1- 6 224 626

## Description

### BACKGROUND OF THE INVENTION

The present invention deals with medical devices. More specifically, the present invention deals with medical devices, such as stents, that can be deployed without undergoing material deformation.

Stents are well known for use in opening and reinforcing the interior wall of blood vessels and other body conduits. Stents are generally tubular, radially expandable and may be of a self-expanding type or can be expandable with an outwardly directed pressure applied to the stent, typically by expansion of an interiorly positioned balloon. Stents are conventionally made of various materials such as plastic or metal.

Conventional stents suffer from a number of disadvantages. One of the problems associated with conventional stents is that current stent designs are limited in the amount of diameter change which can be obtained with the stent as it moves from an unexpanded, insertion position, to an expanded, deployed position. The relative change in diameter of the stent is limited by the characteristics of the material used to make the stent. The combination of materials used to make the stent, and the design of the stent, must be such that the stent can withstand the crimping and expansion thereof, without surpassing its material limits. This restricts the type of materials that can be used.

Recently, work has been done in coating the outside of stents with polymer or ceramic. However, these coatings are prone to cracking as an affect of the crimping and expansion strains when a stent is crimped or expanded.

Other medical devices must also bear strains without deleterious affects. For instance, some medical devices for use in a body cavity, such as integrated electronics or drug containers, can be completely destroyed by large strains.

From US 2002/0188345 A1 an expandable metallic stent is known, having discontinuities of non-conducting material. These eliminate electrically conducting parts in the stent rings and cells so that the stent can be easier imaged with magnetic resonance imaging. The non-conducting materials can include ceramics.

### SUMMARY OF THE INVENTION

Another problem associated with conventional stents involves magnetic resonance imaging (MRI) visualization. MRI visualization is being explored as a visualization technique to be used when implanting stents. However, existing stent designs are incompatible with MRI visualization due to the permanent magnetic disturbance as a result of the magnetic susceptibility of the metals being used as well as the dynamic disturbance of the magnetic field due to Faraday's law as a result of the strong radio frequency (RF) fields and switched gradient magnetic fields in MRI systems in combination with the metallic cage construction of stents. The stent distorts the MRI visualization in an area closely proximate the stent in the anatomy in which it is being implanted. Therefore, some techniques are being explored which involve combining relatively low magnetic susceptibility materials with low susceptibility metals such as titanium or tantalum to create a stent which is more compatible with MRI visualization techniques. Secondly, electrical isolating materials are integrated into metal stent designs in such a pattern that there are no undesirable electrically conducting loops in the structure. Ceramics and polymers are materials which can be used to fulfill the role of the isolator material. However, using a material, such as ceramic, can present its own challenges.

Ceramics are more biocompatible, stronger and more durable than polymers, but are less flexible. Thus, ceramics have a disadvantage in that they perform very poorly in tensile situations. Also, due to their elongation properties, which are virtually non-existent, it is nearly impossible to bend a ceramic. Therefore, if the stent is formed by replacing small parts of the metal structure of the stent by a similar geometry part made out of ceramic (or a similar material), it is desirable that the ceramics be placed in the lowest stress locations in the stent structure.

These types of integrated material stents also suffer from another disadvantage. To remove metal sections out of a finished stent and then to glue ceramic pieces, similar in geometry, into the place where the metal is removed is quite a cumbersome task. It is very difficult to position an extremely small ceramic piece within the complex metal structure of a stent during a bonding operation. Similarly, due to the relatively high number of processing steps needed to produce stents (such as laser cutting, polishing, etc.) tolerance buildup yields variation in the cross-section size of the struts of the stent, which makes it virtually impossible to create exactly matching ceramic pieces. Therefore, using this technique to create a more MRI compatible stent has economic drawbacks, particularly if the process must be repeated up to 10-30 times for every stent.

One embodiment of the present invention thus provides a medical device, such as a stent, made of a relatively inflexible material yet which can still be moved from the crimped or radially contracted insertion position to the radially expanded deployed position. In one embodiment, portions of the stent are made out of relatively inflexible material and are connected together with a hinge connection. This' allows the stent to incorporate materials having relatively low ultimate strain values, such as ceramics, without subjecting these materials to high strain or stress values. This also allows the stent to be assembled on top of a deployment balloon, completely avoiding the crimping process.

In another embodiment, the hinge includes a fixation member that fixes the hinge in a desired deployment position.

In yet another embodiment, not claimed, the stent is provided with sliding elements that allow the stent to expand and contract without stressing the stent material. Thus, the stent can be made of a sheet of rolled material that overlaps itself, and the sliding elements interact so the stent can be expanded to a desired diameter.

In still another embodiment, not claimed, the sheet of rolled material is provided with a mechanical locking mechanism that allows the stent to expand, but precludes it from slipping into a conformation with a smaller radial diameter.

In a further embodiment, not claimed, the sliding elements are deployed on stent struts such that the struts can be transported relative to one another in a longitudinal direction, along a longitudinal axis of the stent.

### BRIEF DESCRIPTION OF THE DRAWINGS

FIG. 1 illustrates a conventional stent.
FIG. 2 illustrates a portion of a strut with hinges in accordance with one embodiment of the present invention.
FIG. 3 illustrates a stent employing the struts shown in FIG. 2.
FIGS. 4A-4D illustrate various hinge embodiments which can be used in accordance with different embodiments of the present invention.
FIGS. 5A-5B illustrate a locking arrangement in which the hinges can be locked in a desired position.
FIGS. 6A-6D illustrate sliding elements disposed within a stent in accordance with one embodiment of the present invention.
FIG. 7 illustrates a mechanical locking arrangement for locking the stent shown in FIG. 6A in an expanded position.
FIGS. 8A-8C illustrate another embodiment of sliding elements on stent struts.
FIGS. 9A-9D illustrate a connector that connects stent struts in a longitudinal transportable way.

### DETAILED DESCRIPTION OF THE ILLUSTRATIVE EMBODIMENTS

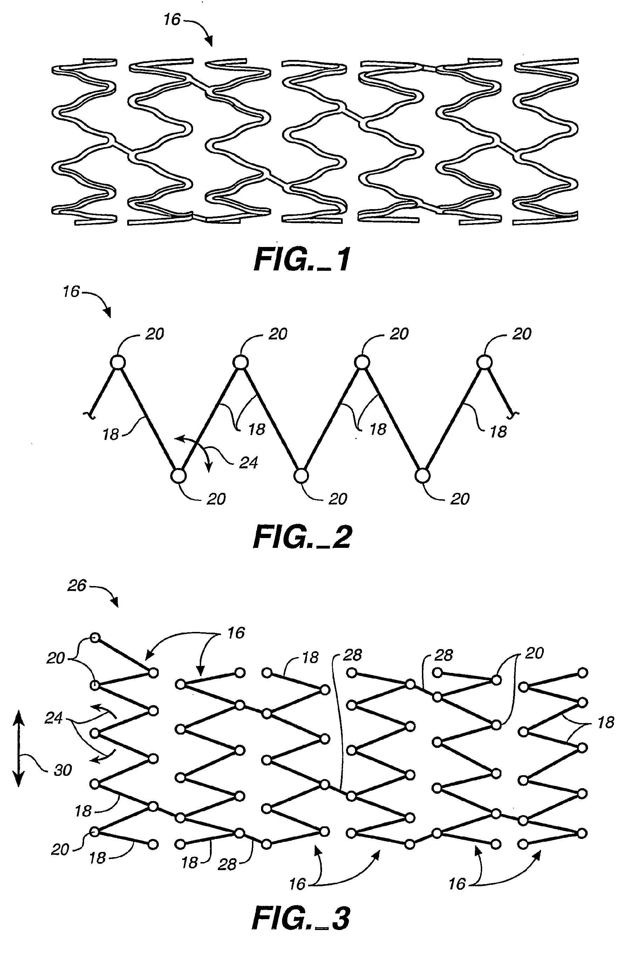

FIG. 1 is a schematic drawing of a segmented stent 10 in accordance with a conventional design. Stent 10 is illustrated as a closed cell design in which a plurality of closed cell stent segments or struts 12 are interconnected by connectors 14. Stent 10, in the past, has been formed as a self-expandable type of stent made of self-expanding material, such as Nitinol. Such stents are cut or etched from tubular stock or rolled or cut or etched from flat sheets of Nitinol or other shape memory metals, which do not themselves exhibit permanent deformation. In general, the self-expanding stent design tends to return to its unconstrained or expanded conformation.

Alternatively, stent 10 has been formed as an expandable stent, which is expandable under an externally applied pressure that is applied to the stent in a radially outward direction. Such stents are typically crimped around an expansion balloon and inserted to a desired position in the vasculature. The balloon is then inflated to drive expansion of the stent.

Both types of prior stent designs have typically been formed of material that has a relatively high magnetic susceptibility causing a significant distortion of the visualization under magnetic resonance imaging (MRI) in the area closely proximate the stent. Furthermore, because of the full metal design of these stents, with highly conductive electrical loops around the cells as well as the circumference of the stent, there is additional distortion of the MRI image due to radio frequency (RF) artifacts caused by both the RF field and gradient magnetic fields in the MRI magnet.

FIG. 2 illustrates a stent strut 16 formed in accordance with one embodiment of the present invention. Stent strut 16 includes strut elements 18 that are connected to one another by hinges 20. The hinges 20 allow stent segments 18 to rotate about hinge points 22, relative to one another, in the direction indicated by arrows 24.

FIG. 3 shows a stent 26 formed of a plurality of struts 16, each with hinges 20 connecting the elements 18. The struts are connected to one another by a plurality of connectors 28. It can thus be seen that, when elements 18 rotate about the hinge points 22 of hinges 20 in the direction indicated by arrows 24, the stent radially expands in the direction indicated by arrow 30. However, when the elements 18 rotate in the opposite direction to that shown by arrows 24, then the stent 26 moves to a radially contracted position, in a direction opposite that shown by arrow 30.

Hinges 20 are illustratively made of any suitable material. Such materials can include, by way of example only, ceramic, polymer, metal or composite, and different parts of the hinges can be made of different materials. For instance, materials can be used to induce a desired friction such as by using ceramic and polymer on male and female portions, respectively, of the hinge. The hinges can also be made of metal with struts and connectors made of ceramic or polymer to avoid electrical loops. Similarly, all parts of the stent can be made of metals, provided with isolating coatings to prevent electrical loops. For example, Teflon or ceramic coatings can be used. Hinges 20 can be made using materials such as ceramic or polymer to a very high precision. Such hinges 20 are illustratively formed using an injection molding process (such as ceramic injection molding-CIM).

Ceramic materials contain many desirable characteristics for producing hinges 20. Ceramic materials are highly durable, have excellent wear resistance, and processes for forming hinges 20 using ceramics are controllable with high precision on the microscopic level. Therefore, several different hinge designs can be readily manufactured.

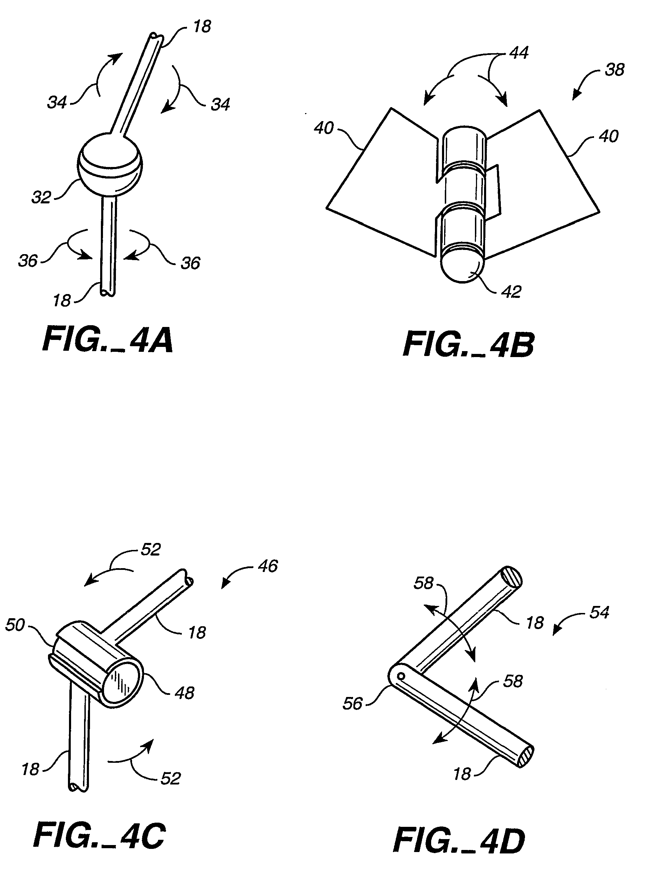

FIGS. 4A-4B illustrate three different types of hinge design. FIG. 4A illustrates a ball and socket hinge 32 which connects elements 18. In one embodiment, the hinge 32 is rotatable about a central pivot point in hinge 32 as is a conventional ball and socket joint. This is indicated by arrow 32. In another embodiment, elements 18 are also rotatable about a longitudinal axis of the elements 18. This is indicated by arrows 36.

FIG. 4B illustrates a leaf hinge 38 which includes leaves 40 connected to one another at a hinge point by a hinge pin 42. The leaves are rotatable about hinge pin 42 in the directions indicated by arrows 44. Leaves 40 are illustratively formed integrally with, or separate from but connected to, elements 18 of struts 16.

FIG. 4C illustrates an elbow hinge 46 in which the hinge point is formed by frictionally snapping an outer sheath attached to a first element 18 over an inner pivot pin 50 that is attached to a second element 18. Of course, the attachments between each of items 48 and 50 and the corresponding elements 18 can be by forming them integrally with one another or by mechanically attaching them to one another. In hinge 46, elements 18 can pivot relative to one another about the pivot point defined by end 46 in the direction indicated by arrows 52.

FIG. 4D illustrates another embodiment of hinge 20 in which the hinge is formed as a knife hinge 54. In knife hinge 54, elements 18 are connected to one another in a side-by-side arrangement and pivot about the pivot point 56 formed by hinge 54 in the direction indicated by arrows 58. Of course, a wide variety of additional hinges can be formed in accordance with the present invention as well.

By providing hinges 20 in stent 26, wherein the hinges are formed of a relatively low magnetic susceptibility material, and of electrical isolating material, electrically conductive loops either about the periphery of stent 26 or about individual cells formed in stent 26, are avoided, as is the permanent magnetic disturbance caused by materials with higher magnetic susceptibility. This significantly reduces the distortion to MRI visualization in the region proximate stent 26. Similarly, by providing hinges 20, the stent material (which may be ceramic, for instance) undergoes low bending stresses during insertion and deployment of the stent.

In addition, because plastic deformation is no longer needed in order to move the stent structure from a crimped position to an expanded position, or vice versa, substantially no portions of the stent 26 undergo high bending or tensional stresses. The radial contraction and expansion movement of the stent is entirely provided for by hinges 20. Therefore, the entire stent 26 can be formed of the relatively low strain materials. Thus, the entire stent 26 can be made of ceramics or ceramic-polymer integrated structures. This can be desirable since ceramics are generally recognized as being more biocompatible than many metals and also because ceramics have a magnetic susceptibility which is near zero.

The arrangement of the stent shown in FIG. 3 also provides additional advantages. It may be desirable in some applications to provide a ceramic coating to metal stent struts. However, the ceramic coatings are prone to cracking during expansion of the stents, because it is difficult for the ceramic coating to follow the same plastic deformation that the metal struts undergo during deployment of the stent. Since stent 26 undergoes radial expansion simply by moving the elements 18 of struts 16, rotating them about hinges 20, a ceramic coating on element 18 does not need to undergo strains induced by following plastic deformation of a metal stent. Thus, ceramic coatings can be used on structural metal elements as well.

In order to assemble hinge 26, a number of different assembly techniques can be used. For instance, stent 26 can be assembled by using elements 18 that are connected to opposing male and female hinge portions at the end of those elements. The male and female hinge portions can be mechanically connected to one another. Alternatively, struts 16 can be fully formed and stent 26 can be assembled by simply using separate connectors 28 to assemble the struts 16 together. Connecting the hinges 20 or connectors 28 allows assembling individual portions of the stent into the overall desired stent conformation. Thus, the stent can be formed on top of a deflated balloon such that hinges 20 are in the collapsed, radially contracted position. This completely avoids the crimping process.

In addition, the configuration of stent 26 shown in FIG. 3 provides additional benefits for drug delivery. For example, assume stent 26 is a drug-coated stent that includes four different struts 16. Also assume that struts 16 are provided with different levels of drug coating thereon, such that they can be selected for assembly based on the level of drugs coated on the struts 16. In one illustrative embodiments, the assembler pre-selects the individual struts 16 out of a large set of struts such that the combined weight of the drug coating is near an ideal weight (or desired drug dosage for the finished stent 26.

By way of example, assume that stent 26 must have a drug load of 100mg of a given drug. Assume that three of the struts 16 have a combined weight of 80mg of drug coating applied to them. The assembler must simply assemble onto the partially assembled stent 26 another strut 16 that has a drug coating weight of 20mg. The assembler can then add to'the assembled stent additional struts with no drug coating, or with different drug coatings thereon.

In order to maintain stent 26 in the radially expanded deployed position, after deployment, or in the radially contracted position during insertion, one of a wide variety of different techniques is illustratively employed. For instance, in one embodiment, the mating hinge surfaces are provided in tight frictional engagement with one another such that it requires a desired amount of force to rotate the hinges. In one embodiment, the mating surfaces of the hinge are simply tightly coupled to one another and are textured to increase the friction between the two during hinge rotation. In another embodiment, the mating hinge portions are threadably engaged to one another such that rotation of the hinge is similar to rotating a screw in a tight fitting bore. In another embodiment, the mating hinge segments are coated with an adhesive to increase the force required to rotate the hinge elements.

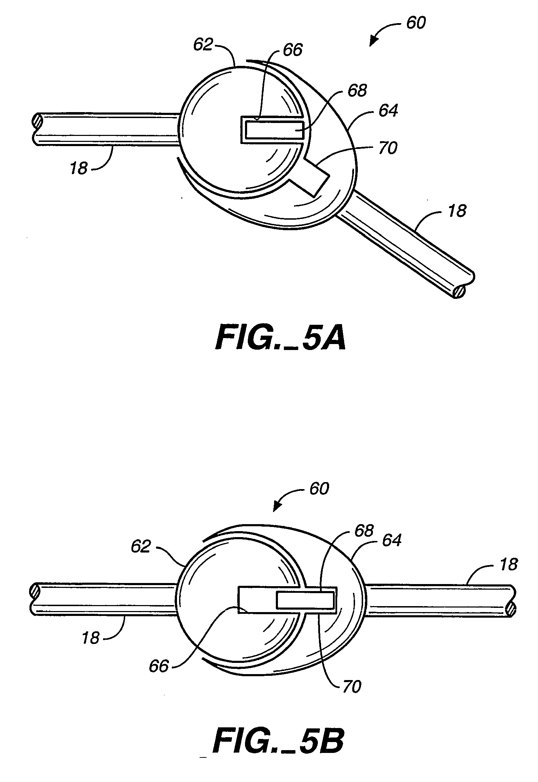

FIGS. 5A and 5B illustrate yet another embodiment of a hinge in which the hinge elements can be maintained in a predetermined orientation relative to one another. FIG. 5A shows hinge 60 that includes a male portion 62 and a female portion 64. Male and female portions 62 and 64 are each connected to stent elements 18, respectively. Male portion 62 has a cavity 66 defined therein with a lug 68 biased in the outward direction contained in cavity 66. The inner bearing race of female portion 64 holds lug 68 within cavity 66 in male portion 62.

However, female portion 64 also has a cavity 70 defined therein. Therefore, as shown in FIG. 5B, when female hinge portion 64 is rotated to a position relative to male hinge portion 62 such that cavity 70 is aligned with cavity 66, the lug 68 exits cavity 66 and is received within cavity 70. This locks the two hinge portions in place relative to one another. The bias force on lug 68 can be provide through any suitable means, such as by using a spring, or such as by deforming lug 68 within cavity 66 and allowing it to assume its relaxed conformation when it is aligned with cavity 70, etc.

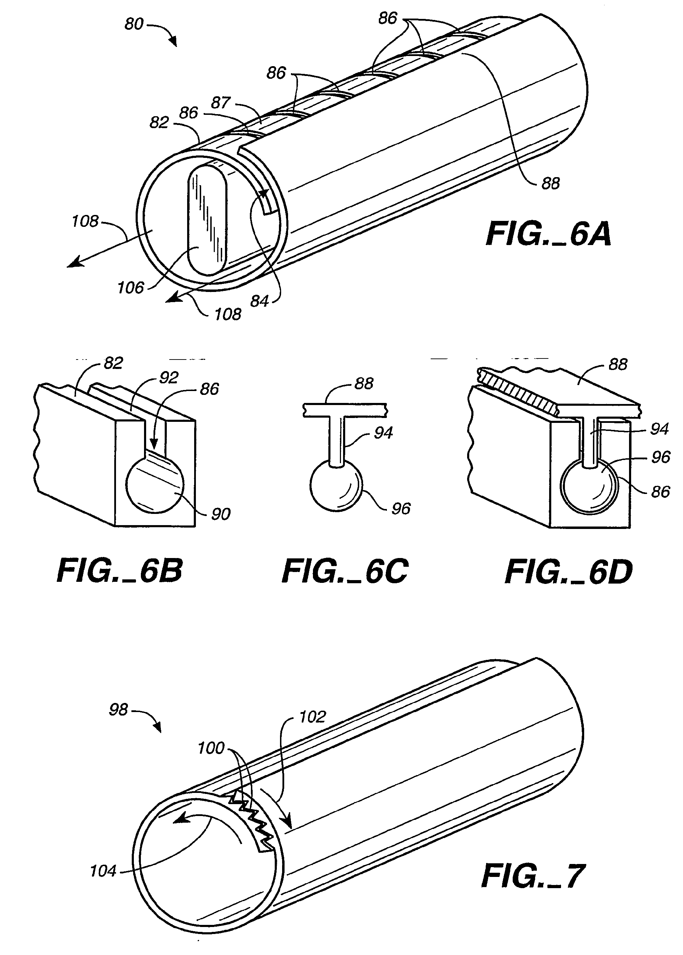

FIGS. 6A and 6B illustrate yet another embodiment of a stent not in accordance with the present invention. FIG. 6A shows a stent 80 that is formed by rolling a sheet 82 of stent material over on itself such that it overlaps in an overlapping region 84. By rolling sheet 82 further upon itself and increasing the length of overlapping region 84, the stent assumes a smaller radial dimension. By unrolling sheet 82 and making overlapping region 84 smaller, stent 80 assumes a larger radial dimension.

In order to provide accurate rolling and unrolling of stent 80 upon itself, sheet 82 has a first portion 87 with slots or grooves 86 formed therein. Sheet 82 also has a second portion, 88, which overlaps the first portion, and has rails or tabs which extend from the overlapping portion 88 down into slots or grooves 86.

FIGS. 6B-6D illustrate this in greater detail. For example, FIG. 6B shows that the portion 87 of sheet 82 that contains grooves 86 has a ball shaped groove section 90 and a channel section 92. The tabs or rails that extend from the second portion 88 of sheet 82 include a tab 94 and ball 96. Ball 96 and tab 94 are sized just small enough to slidably fit within ball shaped opening 90 and channel 92, respectively. Therefore, because ball 96 and tab 94 extend into slot 86, the engagement holds the overlapping portions of sheet 82 closely proximate to one another, yet still allows them to slide relative to one another such that stent 80 can be rolled further onto itself, or unrolled in order to increase its diameter for deployment. FIG. 6D better illustrates that tab 94 and ball 96 are slidably received within slot 86.

FIG. 7 illustrates a fixation mechanism not forming part the invention. that holds stent 80 in the radially expanded, deployed position. In the embodiment, shown in FIG. 7, the portions of sheet 82 that face one another in the overlapping section 84 are each provided with opposing teeth 100. The opposing teeth are arranged such that the two facing portions of sheet 82 can be unfolded relative to one another to a larger diameter in the direction indicated by arrow 102, but they cannot then be slid relative to one another in the direction indicated by arrow 104, to a smaller radial dimension. Thus, during deployment, this locks stent 80 in its radially expanded, deployed position, without allowing it to slip back to a smaller diameter. It should be noted that fixation element 82 can be disposed at a plurality of different locations along the facing regions of sheet 82 that face one another in the overlapping region 84, or it can simply be applied at each end of stent 80, or intermittently there along or continuously there along other than where channels 86 and the associated mating tabs are disposed.

FIG. 6A illustrates yet another embodiment for deploying stent 80. Because the stent is made of a rolled sheet 82, in order to radially expand and deploy stent 80, pressure simply needs to be applied at any point along the interior periphery of stent 80. Therefore, a non-spherical balloon 106 can be used to expand stent 80. For example, FIG. 6A shows that non-spherical balloon 106 is elliptical in shape. This allows the balloon 106 to expand the stent without filling the entire interior of the stent as it is being expanded. Thus, cavities are provided on either side of balloon 106 within stent 80. This allows blood to flow through stent 80, as it is being deployed, as indicated by arrows 108.

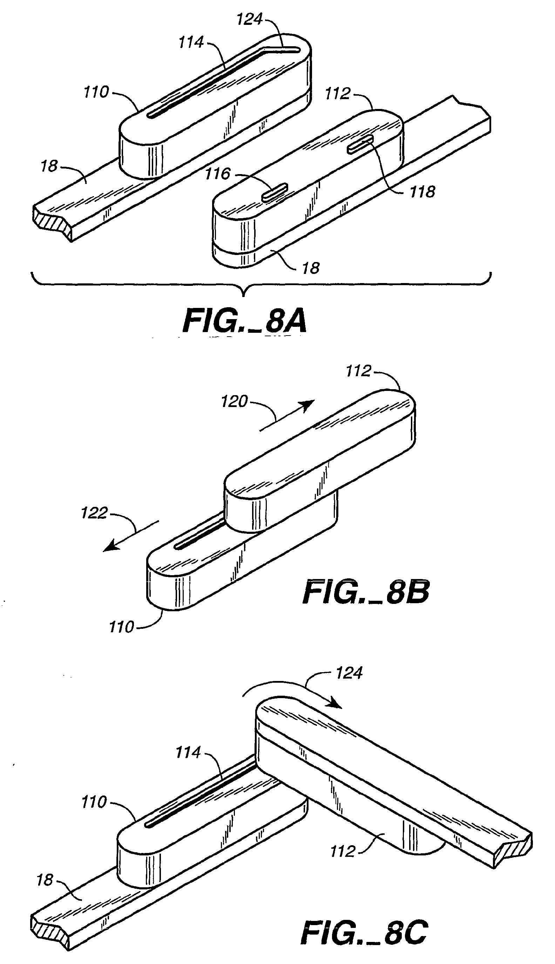

FIGS. 8A-8C illustrate yet another embodiment not forming part of the invention of forming slidable elements relative to one another in order to implement a stent design. In the embodiment shown in FIGS. 8A-8C, individual elements 18 (such as from struts 16 in stent 26 shown in FIG. 3) have, connected thereto, slidable connection members 110 and 112. Connection member 110 has a slot 114 defined therein and connection member 112 has a plurality of tabs 116 and 118 protruding therefrom. In one illustrative embodiment, tabs 116 and 118 are sized just small enough to be slidably received within slot 114. Of course, tabs 116 and 118 and slot 114 can be formed such as that shown in FIGS. 6B and 6C, or in any other suitable configuration. FIG. 8A also shows that elements 18 are connected to elements 110 and 112, respectively, using any suitable connection mechanism, such as adhesive or by forming them integrally with one another, etc.

FIG. 8B shows interaction between connection elements 110 and 112, with elements 18 removed therefrom, simply for the sake of clarity. It can be seen that tab 116 has been inserted within slot 114. Because tab 116 is slidable within slot 114, element 112 can be slid relative to element 110 in the directions indicated by arrows 120 and 122. It can also be seen that slot 114 has a bent region 124. Thus, as sliding movement is continued between elements 112 and 114, tab 116 passes through bent portion 124 in slot 114. This causes element 112 to turn in the direction shown by arrow 124 in FIG. 8C. This causes the corresponding elements 118 to be roved from a positions in which they are generally aligned with one another to a position in which they are unfolded to the configuration shown for example, FIG. 3.

It will, of course, also be understood that elements 18 can, themselves, be formed with slots 114 and tabs 116 and 118. Alternatively, they can be formed separately and connected to one another as shown in FIGS. 8A-8C. In any case, this readily allows the stent to go from a radially contracted position to a radially expanded position simply by sliding the elements relative to one another and locking them in place as shown in the figures.

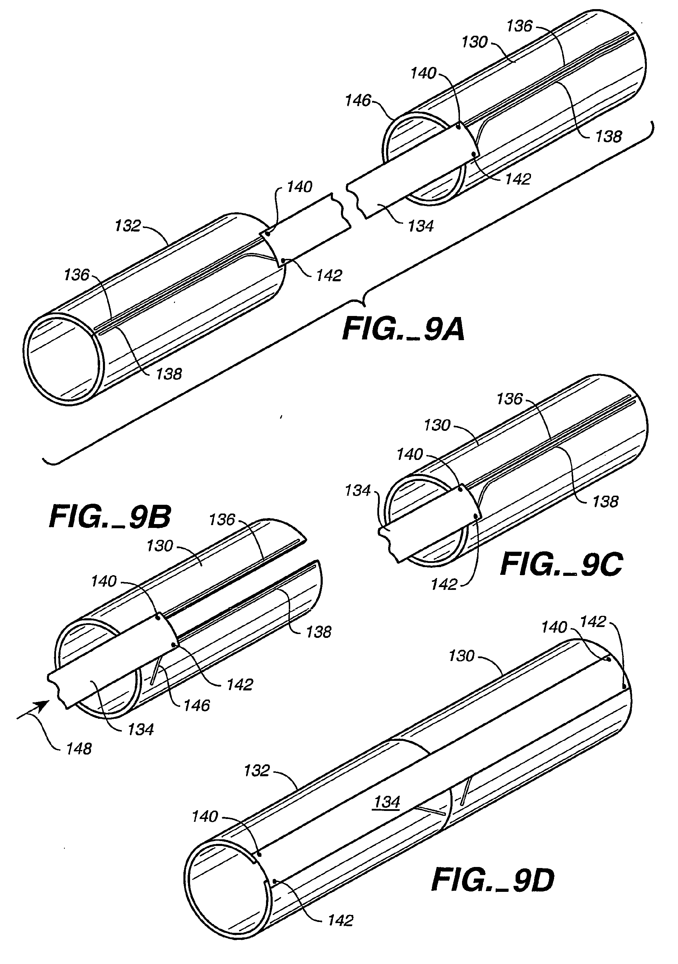

FIGS. 9A-9D illustrate yet another embodiment of a stent not in accordance with the present invention. FIG. 9A shows two stent struts 130 and 132 in a radially contracted position. Stent struts 130 and 132 are connected by a connector 134. Struts 130 and 132 are each formed of rolled sheets of stent material which have, at their facing edges, a plurality of rails 136 and 138, respectively. Connector 134 has a plurality of extending pins 140 and 142, respectively.

FIG. 9B illustrates the rails 136 and 138 and pins 140 and 142 in greater detail. FIG. 9B shows that rail .138 has a bent region 146. Therefore, when pins 140 and 142 are positioned all the way at the end of strut 130, the strut assumes a radially contracted position. However, as connector 134 is advanced in the direction indicated by arrow 148, pins 140 and 142 ride along the rails 136 and 138 respectively. This causes pin 142 to force the stent open to a radially enlarged position as it advances through bent portion 146 of rail 138.

FIG. 9C shows the connector 134 advanced in the direction indicated by arrow 148 past bent region 146 of rail 138. It can be seen that strut 130, in that embodiment, has now assumed a radially expanded conformation. The same configuration is formed with respect to strut 132.

FIG. 9D illustrates strut 130 with connector 134 advanced all the way to the distal end of strut 130. Thus, strut 130 is expanded to the radially expanded position, but the opening therein has been closed by a portion of connector 134. FIG. 9D also shows that the connector has been advanced all the way to the distal end of strut 132. Therefore, struts 130 and 132 are adjacent one another in a radially expanded position.

This provides a number of different advantages. First, it allows transportation of stent struts along the longitudinal axis of the stent. This allows connectors 134 to be made of a relatively flexible material and therefore the stent will be highly flexible when it is in its radially contracted, insertion position such as that shown in FIG. 9A. This is because the struts are less densely packed along the longitudinal axis of the stent when they are separated by connector 134. This also results in higher flexibility during insertion of the stent.

However, when the connectors 134 are advanced within the struts 130 and 132, such that the stent is in its radially expanded deployed position shown in FIG. 9D, it is less flexible. The stent struts 130 and 132 are positioned closely adjacent one another.

The stent embodiment illustrated in FIGS. 6A-9D can illustratively be formed with metal injection molding (MIM) processes. This type of process allows for very fine precision on a very small scale. In addition, these embodiments can also be formed using ceramic injection molding (CIM) which can be used to produce the detailed small-scale ceramic components. Thermoset components can be made using polymer injection methods.

It can thus be seen that the present invention provides for a stent which can be moved between its retracted and expanded positions without requiring plastic or permanent deformation of the stent material. Similarly, the movement between the two positions can be accomplished without imparting a great deal of stress on the stent material, or without requiring any elongation of the stent material.

Thus, the stent can be made of material which does not plastically deform well, such as ceramic. This provides for a high degree of biocompatibility and excellent durability, while enhancing the visualization available through MRI techniques.

Although the present invention has been described with reference to preferred embodiments, workers skilled in the art will recognize that changes may be made in form and detail without departing from the scope of the invention.

## Claims

1. A stent (10) comprising :
a structure forming a generally tubular conformation, the structure formed at least partially of ceramic material and being movable between a contracted position and an expanded position without deformation of the ceramic material, wherein the structure comprises at least one strut (12) that includes a plurality of elements (18), the elements (18) being pivotally connected to one another by a hinge (20) formed of a hinge material,
**characterized in that** the hinge material comprises ceramic.

2. The stent of claim 1 wherein the tubular structure moves between the contracted and expanded positions by pivoting the elements (18) about the hinge (20).

3. The stent of claim 1 wherein the strut (12) is formed of ceramic material.

4. The stent of claim 1 wherein the hinge (20) includes a fixation assembly fixing the elements (18) in a position relative to one another.

5. The stent of claim 1 wherein the structure comprises a plurality of struts (12) with pivotally connected elements (18), the plurality of struts (12) being connected by connectors (18).

6. The stent of claim 1 wherein the structure includes at least one strut (12) formed of connected elements (18).

## Patentansprüche

1. Stent (10), umfassend
eine Struktur, die eine allgemein röhrenförmige Konformation bildet, wobei die Struktur mindestens teilweise aus keramischem Material ausgebildet ist und ohne Verformung des keramischen Materials zwischen einer zusammen gezogenen Position und einer expandierten Position bewegbar ist und die Struktur mindestens eine Strebe (12) umfasst, welche eine Vielzahl von Elementen (18) beinhaltet, wobei die Elemente (18) durch ein aus einem Scharniermaterial gebildetes Scharnier (20) schwenkbar miteinander verbunden sind,
**dadurch gekennzeichnet, dass** das Scharniermaterial Keramik umfasst.

2. Stent gemäß Anspruch 1, wobei die röhrenförmige Struktur sich zwischen der zusammen gezogenen und der expandierten Position bewegt, indem die Elemente (18) um das Scharnier (20) herum geschwenkt werden.

3. Stent gemäß Anspruch 1, wobei die Strebe (12) aus keramischem Material ausgebildet ist.

4. Stent gemäß Anspruch 1, wobei das Scharnier (20) eine Befestigungsbaugruppe beinhaltet, welche die Elemente (18) in Bezug zueinander in einer Position befestigt.

5. Stent gemäß Anspruch 1, wobei die Struktur eine Vielzahl von Streben (12) mit schwenkbar verbundenen Elementen (18) umfasst und die Vielzahl von Streben (12) durch Verbinder (18) verbunden sind.

6. Stent gemäß Anspruch 1, wobei die Struktur mindestens eine aus verbundenen Elementen (18) gebildete Strebe (12) beinhaltet.

## Revendications

1. Stent (10), comprenant
une structure formant une conformation généralement tubulaire, la structure étant formée au moins partiellement de matériau céramique et étant mobile entre une position contractée et une position dilatée sans déformation du matériau céramique, la structure comprenant au moins un étai (12) qui inclût une pluralité de éléments (18), les éléments (18) étant rattachés l'un à l'autre de façon pivotante par une charnière (20) formée par un matériau de charnière,
**caractérisé en ce que** le matériau de charnière comprend de la céramique.

2. Stent selon la revendication 1, dans lequel la structure tubulaire remue entre la position contractée et la position dilatée en pivotant les éléments (18) autour de la charnière (20).

3. Stent selon la revendication 1, dans lequel l'étai (12) est formé de matériau céramique.

4. Stent selon la revendication 1, dans lequel la charnière (20) inclût un ensemble de fixation qui fixe les éléments (18) dans une position l'un par rapport à l'autre.

5. Stent selon la revendication 1, dans lequel la structure comprend une pluralité d'étains (12) avec des éléments (18) rattachés de façon pivotante et dans lequel la pluralité d'étains (12) sont rattachés par des connecteurs (18).

6. Stent selon la revendication 1, dans lequel la structure inclût au moins un étai (12) formé par des éléments (18) rattachés.
